(19) Europäisches Patentamt
European Patent Office
Office européen des brevets

(11) Publication number: **0 086 292**
**B1**

# (12) EUROPEAN PATENT SPECIFICATION

(45) Date of publication of patent specification: **23.04.86**

(51) Int. Cl.[4]: **B 01 J 23/88,** C 07 C 5/48, C 07 C 45/34, C 07 C 51/00, C 07 C 120/14

(21) Application number: **82300744.8**

(22) Date of filing: **15.02.82**

## (54) Production of catalysts by impregnation and use thereof in oxidation type reactions.

(43) Date of publication of application:
**24.08.83 Bulletin 83/34**

(45) Publication of the grant of the patent:
**23.04.86 Bulletin 86/17**

(84) Designated Contracting States:
**BE CH DE FR GB IT LI LU NL SE**

(56) References cited:
**DE-A-2 157 402**
**FR-A-2 348 741**
**GB-A-1 478 621**
**US-A-3 415 886**
**US-A-3 642 930**
**US-A-4 182 907**

(73) Proprietor: **THE STANDARD OIL COMPANY**
**Midland Building**
**Cleveland, Ohio 44115 (US)**

(72) Inventor: **Friedrich, Maria Strada**
**5686 Ridgebury Boulevard**
**Lyndhurst Ohio 44124 (US)**
Inventor: **Grasselli, Robert Karl**
**150 Greentree Road**
**Chagrin Falls Ohio 44022 (US)**
Inventor: **Suresh, Dev Dhanaraj**
**1052 Iroquois Run**
**Macedonia Ohio 44056 (US)**

(74) Representative: **Smith, Sydney et al**
**Elkington and Fife High Holborn House 52/54**
**High Holborn**
**London WC1V 6SH (GB)**

**EP 0 086 292 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to improved oxide complex catalysts for use in various oxidation-type reactions such as, for example, the oxidation of propylene or isobutylene to produce acrolein and acrylic acid or methacrolein and methacrylic acid respectively, the ammoxidation of propylene or isobutylene to produce acrylonitrile or methacrylonitrile, respectively, or the oxydehydrogenation of isoamylenes to produce isoprene.

Iron bismuth molybdate oxidation catalysts are well known and many different promoters have been proposed for such systems. See, for example, U.S. Patent 3,642,930, in which alkali metals and optionally cobalt and/or nickel are disclosed as useful. See also U.S. Patent 4,123,453 wherein the Group II elements are proposed.

Although known catalysts exhibit good catalytic properties, it is a constant objective to provide new catalysts having even better catalytic properties.

Accordingly, it is an object of the present invention to provide a new technique for making catalysts of improved catalytic properties.

We have found that iron bismuth molybdate oxide complex catalysts promoted with Group I, II and/or III elements of improved catalytic performance can be obtained provided that at least some of the Group I, IIB or III elements are incorporated into the catalyst by impregnation of a preformed and calcined iron bismuth molybdate oxide complex.

Thus, the present invention provides an improvement in the known process for producing an oxidation catalyst comprising a substantially crystalline iron bismuth molybdate oxide complex catalyst wherein at least a portion of one or more elements in the catalyst selected from Groups IA, IB, IIB, IIIA and IIIB of the Periodic Table is incorporated into the catalyst by impregnation and further wherein none of the Group IA elements incorporated into the catalyst by impregnation is derived from a molybdate or silicate.

Catalysts to which the present invention applies (i.e. final catalyst product) are substantially crystalline oxide complexes having the following general formula:

$$A_aB_bC_cBi_dFe_eMo_fO_x$$

wherein

- A is at least one element selected from the Group I, II or III elements;
- B is one or more Group VIII elements other than Fe, preferably Co and/or Ni and optionally Cr and/or Mn;
- C is P, As, Sb, S, Te and/or Sn;

and wherein

- a is 0.1—12;
- b is 0.1—12;
- c is 0.1—12;
- d is greater than 0—12;
- e is 0.1—12;
- f is 0.1—12; and
- x is a number determined by the valence requirements of the other elements present;

and further wherein the stoichiometric amount of molybdenum content is greater than the content of any other element in the catalyst, excluding oxygen.

Preferably, a is 0.1 to 6, b is 0.1 to 12, c is 0.1 to 12, d is greater than 0 to 4, e is 0.1 to 12 and f is 12.

The above catalysts normally contain at least some alkali metal. Also, the bismuth content is usually 0.1 to 5, preferably 0.5 to 2, more preferably about 1, based on 12 atoms of molybdenum. In addition, the iron content is normally about 1 to 5, preferably 1.5 to 4, more preferably 2 to 3, based on 12 atoms of molybdenum.

Especially preferred catalysts within the above definition are those defined by the following formula:

$$I_mII_nIII_oB_pD_qFe_rBi_sMo_tO_x$$

wherein

- I is at least one Group I element, preferably K, Rb, Cs and/or Cu;
- II is at least one Group II element, preferably Mg, Ca and/or Zn;
- III is at least one Group III element, preferably Al and/or Tl;
- B is at least one of Co, Ni and optionally Mn and/or Cr;
- D is P, As, Sb, Te and/or Sn;

and wherein

- m is 0 to 1;
- n is 0 to 12;
- o is 0 to 12;
- p is 0 to 10;
- q is 0 to 6;
- r is 1 to 1/2 t;
- s is 0.5 to 1/2 t;
- t is 8 to 16;
- x is a number determined by the valence requirements of the other elements present.

The catalysts of the invention can be either unsupported or supported. If supported, conventional supports such as silica, alumina, Alundum®, zirconia, titania and the like can be employed. Any amount of support can be employed.

The catalysts of the present invention are made by forming an oxide complex interim catalyst in a conventional manner except that at least some of the Group I, IIB and/or III elements of the catalysts are added by an impregnation technique using a solution of these elements to impregnate a preformed and calcined oxide complex interim catalyst of the other elements. Thus in accordance with the present invention, an oxide complex interim catalyst is formed by a conventional

technique, this oxide complex interim catalyst containing all of the elements of the desired catalyst except for at least some of the Group I, IIB and/or III elements which are to be added by subsequent impregnation. Thus after the oxide complex interim catalyst is made, it is impregnated with a solution of a Group I, IIB and/or III element, dried and calcined to produce the objective catalyst.

In accordance with the first step of the present invention therefore, an oxide complex interim catalyst is formed by a conventional technique. Most easily, this is accomplished by forming a solution or slurry, usually in water, of the elements to be incorporated into the oxide complex interim catalyst usually in the form of salts having heat decomposable anions or cations and/or oxides. The liquid slurry medium is removed from the slurry to form a precatalyst and the precatalyst is then calcined in air at elevated temperature, for example 600 to 900°C for an extended period of time, 0.5 to 50 hours. During such calcination, care should be taken to avoid gross decomposition of the catalyst such as occurs in McClellan, U.S. Patent 3,415,886.

In this regard, an essential feature of the McClellan technique is that the interim bismuth molybdate on silica material which is composed of various crystalline molybdate be heated under conditions which are so severe that these crystalline materials are substantially destroyed and replaced with a substantially amorphous phase. This procedure is totally avoided in accordance with the present invention whereby the catalysts produced have the conventional structure, i.e. they are substantially crystalline. Techniques for making oxide complexes in the conventional manner are well known and disclosed, for example, in U.S. Patent 3,642,930.

In another embodiment of the invention, the oxide complex interim catalyst can be a used catalyst, that is a catalyst which has already been used in an oxidation or ammoxidation type reaction to produce valuable product.

Once the oxide complex interim catalyst is produced as described above, it is impregnated in accordance with the present invention with a solution of a Group I, IIB or III element. In order to obtain dissolution of the Group I, IIB or III element, a compound of the element which is soluble in the liquid used to form the slurry is normally employed. For example, if water is chosen as the solution medium, nitrates of the elements can be used. On the other hand, if an organic liquid such as an alcohol is used as the liquid, organic compounds of the elements in question, such as for example acetates can be employed. Also, it is possible to use the element directly if they can be made to dissolve in the appropriate liquid.

In our U.S. Patent 3,280,166 there is shown improvement of the properties of a bismuth molybdate or bismuth phosphomolybdate catalyst by impregnating the catalysts with materials which decompose to yield barium oxide and silicon oxide. Potassium silicate is an example of a material which will supply silicon for the silicon oxide. U.S. 4,052,332, discloses a process for rejuvenating used or spent iron bismuth molybdates in which the spent catalyst is impregnated with a solution containing bismuth and molybdenum. Alkali metal molybdates are examples of materials which can be used to supply molybdenum to the impregnating solution. In accordance with both these techniques, alkali metal would be deposited on the catalyst if a silicate or molybdate of an alkali metal were employed as a source compound, and therefore in accordance with the present invention silicates or molybdates of alkali metal are not employed as source compounds for supplying alkali metal to the impregnating solution.

The liquid used for the impregnating solution is not critical and both aqueous and organic liquids can be employed. For example, in addition to water, various alcohols and especially ethanol, propanol and so forth can be employed as can acetone, hydrocarbons, etc. Also, mixed systems can be employed. For example, solutions of water and various organic acids such as tartaric, oxalic, acetic, citric, chloroacetic acid and the like can be employed. Also, various compounds which aid solubility can also be included. For example, various mineral acids such as $HNO_3$, $H_2SO_4$, HCl, $H_3PO_4$ and the like can be included. Basically, any liquid can be employed so long as the liquid does not exhibit a significant adverse effect on the ultimate catalyst produced. For example, strong bases should be avoided since they may poison the ultimate catalyst product.

The impregnating element is deposited in the oxide complex interim catalyst by mixing therewith the impregnating solution and thereafter removing the liquid. For ease of operation and control of the amount of impregnant deposited on the catalyst, this is usually accomplished by mixing the impregnating solution and the oxide complex interim catalyst together and thereafter evaporating the impregnating liquid.

The concentration of impregnant in the impregnating liquid and the relative amounts of impregnating liquid and oxide complex interim catalyst are not critical and can be easily selected by those skilled in the art to facilitate ease of impregnation. Usually, however, the amount of impregnating solution will be no more than 1.5, preferably 1.1, more preferably 1.0 times the pore volume of the interim catalyst to be impregnated.

Once the impregnating liquid is removed, the impregnated oxide complex interim catalyst is calcined at elevated temperature and pressure to set the impregnant metal into the catalyst and establish the final relationship of the atoms therein. Calcination is accomplished by heating at elevated temperature in the presence of an oxygen-containing gas, normally air, in a routine manner. For example, heating in accordance with the above-noted U.S. Patent 3,642,930, is preferred. Again, care must be taken to avoid

destruction of the crystalline bismuth molybdates as occurs in the above-noted McClellan patent.

The catalysts produced by the process of the invention can be used in all oxidation-type reactions in which analogous catalysts are used, such as for example oxidation of olefins to produce aldehydes and acids, ammoxidation of olefins to produce unsaturated nitriles and oxydehydrogenation of olefins to produce diolefins.

The following examples illustrate the present invention.

Comparative Example A

A silica-supported, alkali metal-promoted catalyst of the type shown in U.S. 3,642,930 was charged into a fluid-bed reactor and contacted with a feed comprising

1.8 propylene/2.2 $NH_3$/3.6 $O_2$/2.4 $N_2$/6 $H_2O$

at 430°C for a contact time of 2 seconds. The gross reaction product obtained was recovered and analyzed and it was found that acrylonitrile was produced in yields of 77.6% based on the propylene fed with a selectivity to acrylonitrile of 82.2%.

Example 1

Comparative Example A was repeated except that 25 gms. of the catalyst of Comparative Example A after being formed was impregnated with 20 gms. of an aqueous solution containing 0.042 gms. potassium acetate. After impregnation, the catalyst was washed with water, dried and then calcined in air at 570°C for 3 hours. The potassium-impregnated catalyst was then used in the same way as in Comparative Example A to produce acrylonitrile. In this example, acrylonitrile was produced with yields of 80.0% based on the propylene fed with a selectivity to acrylonitrile of 81.8%.

Example 2

Example 1 was repeated except that the impregnating solution was composed of an aqueous solution of copper acetate, and the amount of copper impregnated in the catalyst was such that the copper content was $Cu_{0.2}$ based on 12 molybdenum atoms. In this example, acrylonitrile was produced with yields of 79.4% based on the propylene fed with a selectivity to acrylonitrile of 81.8%.

From the above it can be seen that catalysts of improved properties can be produced by forming the catalysts using an impregnating technique to incorporate at least some of the Group I, IIB or III elements into the catalyst.

**Claims**

1. A process for improving the catalytic properties of a substantially crystalline iron bismuth molybdate oxide complex interim catalyst comprising incorporating at least one element from Groups IA, IB, IIB, IIIA and IIIB of the Periodic Table into the interim catalyst by impregnation, none of the Group IA elements incorporated into the interim catalyst by impregnation being derived from a molybdate or silicate, to produce a catalyst of the formula:

$$A_aB_bC_cBi_dFe_eMo_fO_x$$

wherein
A is at least one element selected from the Group I, II and III elements;
B is one or more Group VIII elements other than Fe, preferably Co and/or Ni and optionally Cr and/or Mn;
C is P, As, Sb, S, Te and/or Sn;

and wherein
a is 0.1—12;
b is 0.1—12;
c is 0.1—12;
d is greater than 0—12;
e is 0.1—12;
f is 0.1—12; and
x is a number determined by the valence requirements of the other elements present;

and further wherein the stoichiometric amount of molybdenum is greater than the content of any other element in the catalyst excluding oxygen.

2. A process as claimed in claim 1 characterised in that the oxide complex interim catalyst is formed by forming a precatalyst and thereafter calcining said precatalyst in an oxygen containing gas, (b) the oxide complex interim catalyst is impregnated with a solution of said element, and (c) the impregnated oxide complex interim catalyst is calcined in an oxygen containing gas.

3. A process as claimed in claim 2 characterised in that the solution is free of molybdenum and silicon.

4. A process for the catalytic oxidation of olefins to produce aldehydes and acids, ammoxidation of olefins to produce unsaturated nitriles and oxydehydrogenation of olefins to produce diolefins, wherein a catalyst produced by a process as claimed in any of claims 1 to 3 is used.

**Patentansprüche**

1. Verfahren zum Verbessern der katalytischen Eigenschaften eines im wesentlichen kristallinen Eisen - Wismuth - Molybdat - Oxid - Komplex - Zwischenkatalysators, bei dem wenigstens ein Element aus der Gruppe IA, IB, IIB, IIIA und IIIB des Periodischen Systems durch Imprägnieren in den Zwischenkatalysator eingefügt wird, wobei keines der durch Imprägnieren in den Zwischenkatalysator eingegebenen Elemente der Gruppe IA von Molybdat oder Silikat abgeleitet ist, um einen Katalysator der Formel zu erzeugen:

$$A_aB_bC_cBi_dFe_eMo_fO_x,$$

wobei

A wenigstens ein Element aus der Gruppe I, II und III Elemente;
B eines oder mehrere der Gruppe VIII Elemente mit Ausnahme von Fe, vorzugsweise Co und/oder Ni und wahlweise Cr und/oder Mn;
C P, AS, SB, S, Te und/oder Sn ist;

und wobei
a=0.1—12;
b=0,1—12;
c=0,1—12;
d größer als 0—12;
e=0,1—12;
f=0,1—12; und
x eine von den Wertigkeitsanforderungen der anderen vorhandenen Elemente bestimmte Zahl ist;

und wobei weiterhin die stöchiometrische Menge des Molybdens größer als der Gehalt jedes anderen Elements im Katalysator mit Ausnahme von Sauerstoff ist.

2. Verfahren nach Anspruch 1, dadurch gekennzeichnet, daß a) der Oxid-Komplex-Zwischenkatalysator durch Bilden eines Vorkatalysators und durch anschließendes Kalzinieren des Vorkatalysators in einem sauerstoffhaltigen Gas gebildet wird, b) der Oxid-Komplex-Zwischenkatalysator mit einer Lösung des besagten Elementes imprägniert wird und c) der imprägnierte Oxid-Komplex-Zwischenkatalysator in einem sauerstoffhaltigen Gas kalziniert wird.

3. Verfahren nach Anspruch 2, dadurch gekennzeichnet, daß die Lösung kein Molybden und kein Silikon enthält.

4. Verfahren zum katalytischen Oxidieren von Olefinen zum Erzeugen von Aldehyden und Säuren, zu Amoxidation von Olefinen, zum Erzeugen ungesättigter Nitrile und zur Oxydehydrogenisierung von Olefinen, zum Erzeugen von Diolefine, wobei ein nach einem Verfahren gemäß einem der Ansprüche 1 bis 3 hergestellter Katalysator benutzt wird.

**Revendications**

1. Procédé pour améliorer les propriétés catalytiques d'un catalyseur intérimaire complexe oxydé molybdate de fer et de bismuth en grande partie cristallin consistant à incorporer au moins un élément des groupes IA, IB, IIB, IIIA et IIIB de la classification périodique dans le catalyseur intérimaire par imprégnation, aucun des éléments du groupe IA incorporé dans le catalyseur intérimaire par imprégnation ne provenant d'un molybdate ou d'un silicate, pour produire un catalyseur répondant à la formule:

$$A_aB_bC_cBi_dFe_eMo_fO_x$$

dans laquelle
A est au moins un élément choisi parmi les éléments des groupes I, II et III;
B est un ou plusieurs des éléments du groupe VIII autre que Fe, de préférence Co et/ou Ni et, éventuellement, Cr et/ou Mn;
C est P, As, Sb, S, Te et/ou Sn;

et dans laquelle
a est 0,1—12;
b est 0,1—12;
c est 0,1—12;
d est supérieur à 0—12;
e est 0,1—12;
f est 0,1—12; et
x est un nombre déterminé par les exigences de valence des autres éléments présents;

et en outre dans laquelle la quantité stoechiométrique de molybdène est supérieure, à la teneur en tout autre élément du catalyseur à l'exception de l'oxygène.

2. Procédé selon la revendication 1, caractérisé en ce que (a) le catalyseur intérimaire complexe oxydé est formé en formant un précatalyseur puis en calcinant ce précatalyseur dans un gaz contenant de l'oxygène, (b) le catalyseur intérimaire complexe oxydé est imprégné d'une solution dudit élément, et (c) le catalyseur intérimaire complexe oxydé imprégné est calciné dans un gaz contenant de l'oxygène.

3. Procédé selon la revendication 2, caractérisé en ce que la solution est exempte de molybdène et de silicium.

4. Procédé pour l'oxydation catalytique des oléfines pour produire des aldéhydes et des acides, ammoxydation des oléfines pour produire des nitriles insaturés et oxydéshydrogénation des oléfines pour produire des dioléfines, dans lequel on utilise un catalyseur préparé par un procédé selon l'une quelconque des revendications 1 à 3.